(19) Europäisches Patentamt
European Patent Office
Office européen des brevets

(11) EP 1 617 219 A1

(12) **EUROPEAN PATENT APPLICATION**
published in accordance with Art. 158(3) EPC

(43) Date of publication:
**18.01.2006 Bulletin 2006/03**

(21) Application number: **04728032.6**

(22) Date of filing: **16.04.2004**

(51) Int Cl.:
***G01N 33/547*** *(1985.01)* ***G01N 33/543*** *(1985.01)*
***C12N 15/00*** *(1980.01)* ***C12Q 1/68*** *(1980.01)*

(86) International application number:
**PCT/JP2004/005498**

(87) International publication number:
**WO 2004/092731 (28.10.2004 Gazette 2004/44)**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **18.04.2003 JP 2003114411**

(71) Applicant: **Nisshinbo Industries, Inc.**
**Chuo-ku,**
**Tokyo 103-8650 (JP)**

(72) Inventors:
- **KIMURA, Naoki,**
**Nisshinbo Industries, Inc.**
**Chiba-shi,**
**Chiba 2670056 (JP)**
- **ODA, Ryuichi,**
**Nisshinbo Industries, Inc.**
**Chiba-shi,**
**hiba 2670056 (JP)**
- **MASUDA, Gen,**
**Nisshinbo Industries, Inc.**
**Chiba-shi,**
**hiba 2670056 (JP)**
- **HASHIBA, Toshifumi,**
**Nisshinbo Industries, Inc.**
**Chiba-shi,**
**hiba 2670056 (JP)**
- **HAYAKAWA, Kazutoshi,**
**Nisshinbo Industries, Inc.**
**Chiba-shi,**
**hiba 2670056 (JP)**

(74) Representative: **Tombling, Adrian George et al**
**Withers & Rogers LLP**
**Goldings House,**
**2 Hays Lane**
**London SE1 2HW (GB)**

# (54) ELEMENT HAVING BIOACTIVE SUBSTANCE FIXED THERETO

(57) By using a biologically active substance-immobilized device, which comprises a base particle comprising a core particle and an organic compound having two or more hydrophilic groups and immobilized on the core particle by a chemical bond and a biologically active substance bonded to the base particle via the organic compound, a substance specifically bonding to the biologically active substance is detected or measured.

EP 1 617 219 A1

SEQUENCE LISTING

<110> Nisshinbo Industries, Inc.

<120> Element Having Bioactive Substance Fixed Thereto

<130> F2293-C4040

<140> JP 2003-114411
<141> 2003-04-18

<160> 4

<170> PatentIn version 3.0

<210> 1
<211> 30
<212> DNA
<213> Artificial/Unknown

<220>
<223> Description of Artificial Sequence: synthetic oligonucleotide

<400> 1
tttttttttt aaatgggtac tgtgcctgtt 30

<210> 2
<211> 30
<212> DNA
<213> Artificial/Unknown

<220>
<223> Description of Artificial Sequence: synthetic oligonucleotide

<400> 2
tttttttttt acgcatccag ctctgaatcc 30

<210> 3
<211> 30
<212> DNA
<213> Artificial/Unknown

<220>
<223> Description of Artificial Sequence: synthetic oligonucleotide

<400> 3

```
tttttttttt acgcatccgg ctctgaatcc                                       30

<210>  4
<211>  262
<212>  DNA
<213>  Artificial/Unknown

<220>
<223>  Description of Artificial Sequence: probe

<400>  4
tcgcccgctg tttttgatga ggcggatttt ccggcagttg ccgtttatct caccggcgct   60
gaatacacgg gcgaagagct ggacagcgat acctggcagg cggagctgca tatcgaagtt  120
ttcctgcctg ctcaggtgcc ggattcagag ctggatgcgt ggatggagtc ccggatttat  180
ccggtgatga gcgatatccc ggcactgtca gatttgatca ccagtatggt ggccagcggc  240
tatgactacc ggcgcgacga tg                                           262
```

## Description

Technical Field

The present invention relates to a device for detection or measurement of a biologically active substance or for therapeutic treatment. The device of the present invention relates to the fields of diagnosis, therapeutic treatment, biochemistry and so forth.

Background Art

In analyses of nucleic acids based on hybridization, immunoassays and so forth, techniques of immobilizing nucleic acids or proteins on a carrier such as particles, membranes and plates have conventionally been utilized. As such methods for immobilizing biomolecules, the following methods are known for nucleic acids:

(1) A method of chemically bonding a nucleic acid introduced with a modification group, such as immobilization by a disulfide bond between a nucleic acid having a thiol group at the 5' end and a bead-like base material having thiol groups (P.J.R. Day et al., Biochem. J., vol. 278, pp.735-740 (1991));
(2) A method of immobilizing a nucleic acid by adsorption on a carrier such as nitrocellulose, nylon membrane, or glass coated with a cationic polymer such as poly-L-Lysine through ultraviolet (UV) irradiation or heat treatment (International Patent Publication in Japanese (Kohyo) No. 10-503841; J. Sambrook et al., Molecular Cloning, Cold Spring Harbor Laboratory Press, Second Edition, pp.2.109-2.113 and pp.9.34-9.46) ;
(3) A method of physically adsorbing a nucleic acid on wells of a microplate treated with a polylysine solution by injecting the nucleic acid into the wells and heating the plate at 37°C (G.C.N. Parry et al., Biochem. Soc. Trans., vol. 17, pp.230-231 (1989));
(4) A method of synthesizing DNA on a base material by using nucleotides bonded to the base material (WO97/10365);
(5) A method of chemically bonding a nucleic acid introduced with a modification group such as immobilization of a nucleic acid having a biotin group at the 5' end on a magnetic bead carrier covered with a streptavidin-coated film (International Patent Publication in Japanese No. 2000-507806); and
(6) A method of immobilizing a nucleic acid on polystyrene beads coated with polycarbodiimide (carbodiimide groups) (Japanese Patent Laid-open (Kokai) No. 8-23975).

However, these methods suffer from drawbacks. That is, the method of (1) requires an extremely special apparatus and regents. Further, in the methods of (2) and (3), nucleic acids are dropped off from the carriers during the hybridization, in particular, in operation processes, and as a result, detection sensitivity may be reduced, or reproducibility cannot be obtained. Furthermore, with these methods, although a long nucleic acid can be immobilized, a short nucleic acid of about 50-mer or shorter such as oligomers cannot be efficiently immobilized. Further, the method of (4) also requires an extremely special apparatus and regents for synthesizing DNA on the base material, and the nucleic acid that can be synthesized is limited to about 25-mer or shorter. Moreover, the method of (5) has drawbacks that the material of the base material is limited, and storage stability of the nucleic acid-immobilized beads is poor. In the method of (6), a nucleic acid is reacted with a carbodiimide group, and therefore the nucleic acid is not separated from the polycarbodiimide during hybridization. However, because the polystyrene and the polycarbodiimide do not bond to each other with chemical bonds, the polycarbodiimide tends to separate from the polystyrene beads during hybridization.
On the other hand, there have been conducted researches in which it is attempted to activate a monocarbodiimide and immobilize a substance on a particle serving as a base material via an amide bond from an amino acid or the like and thereby improve dispersibility of the particles, as described in Japanese Patent No. 2629909. However, because unnecessary products such as urea derivatives are produced by the condensation reaction, a problem of time-consuming washing step arises. Other problems also arise, for example, the substance to be immobilized is limited to particular active substances capable of forming an amide bond, and the product may not exhibit performance as a test device or diagnostic device depending on the bonding site (position) of particle serving as a base material and amino group or carboxyl group in an amino acid.

Disclosure of the Invention

An object of the present invention is to provide a device for detection or measurement of a biologically active substance or for therapeutic treatment, which exhibits favorable stability in a dispersion medium, and a production method thereof.
The inventors of the present invention conducted various researches in order to achieve the aforementioned object. As a result, they found that, by immobilizing a biologically active substance on particles using an organic compound having two or more hydrophilic groups, dispersion stability of the particles in a solution could be improved, and thus accomplished the present invention.

That is, the present invention provides the followings.

(1) A biologically active substance-immobilized device, which comprises a base particle comprising a core particle and an organic compound having two or more hydrophilic groups and immobilized on the core particle by a chemical bond and a biologically active substance bonded to the base particle via the organic compound.
(2) The device according to (1), which is used in an aqueous medium.
(3) The device according to (1) or (2), wherein the base particle has an average particle diameter of 0.01 to 100 μm.
(4) The device according to any one of (1) to (3), wherein the base particle has a spherical or substantially spherical shape.
(5) The device according to any one of (1) to (4), wherein at least one of $CV_b$ ratio and $CV_c$ ratio defined by the following equations is 0.6 to 3.0:

$$CV_b \text{ ratio} = CV_1/CV_3$$

$$CV_c \text{ ratio} = CV_2/CV_3$$

$$CV_1 = (\text{Standard deviation of core particle diameter/Average core particle diameter}) \times 100$$

$$CV_2 = (\text{Standard deviation of base particle diameter/Average base particle diameter}) \times 100$$

$$CV_3 = (\text{Standard deviation of device diameter/average device particle diameter}) \times 100$$

(6) The device according to any one of (1) to (5), wherein the core particle and the biologically active substance are bonded by a reaction with a functional group selected from carbodiimide group, ester group, carbonate group, epoxy group and oxazoline group.
(7) The device according to any one of (1) to (6), wherein the organic compound is a compound represented by the following formula:

$$A_x\text{-}(R\text{-}X)_n\text{-}R\text{-}A_y \qquad (I)$$

wherein $A_x$ and $A_y$ independently represent a segment having a functional group that exhibits hydrophilicity and may be identical or different, R independently represents an organic group of two or more valences, X independently represents carbodiimide group, epoxy group or oxazoline group, and n is an integer of 2 to 80, preferably 2 to 40.
(8) The device according to any one of (1) to (7), wherein the biologically active substance is selected from a nucleic acid, protein, hapten and saccharide.
(9) The device according to any one of (1) to (8), which is for detecting or measuring a second biologically active substance contained in a sample by using a specific bond of the biologically active substance and the second biologically active substance in the sample.
(10) The device according to any one of (1) to (8), wherein the biologically active substance is an agent for therapeutic treatment of a disease.

Best Mode for Carrying out the Invention

Hereafter, the present invention will be explained in detail.

<1> Device of the present invention

The device of the present invention comprises a base particle comprising a core particle and an organic compound having two or more hydrophilic groups (hereinafter referred to as "organic compound A") immobilized on the core particle by a chemical bond and a biologically active substance bonded to the base particle via the organic compound A.
Hereafter, the device of the present invention will be explained.

(1) Core particle

The core particle serves as a support on which a biologically active substance is to be immobilized. According to one embodiment of the device of the present invention (affinity particle), the "biologically active substance" is for capturing a second biologically active substance in a sample by a specific bond of the biologically active substance and the second biologically active substance. Examples of the sample include body fluids such as blood, plasma and serum, cells such as animal or plant cells and bacteria and so forth.
According to another embodiment of the device of the present invention, the biologically active substance acts as an agent used for therapeutic treatment (active ingredient) or as a ligand for bonding the agent. Examples of the biologically active substance include nucleic acids such as DNA and RNA, proteins (including peptides) such as antigens, antibodies and enzymes, peptide nucleic acids, haptens, saccharides, glycopeptides and so forth. Among these, nucleic acids are preferred. The biologically active substance will be described later.
The aforementioned core particle is preferably insoluble in an aqueous medium and preferably exhibits good dispersibility in an aqueous medium. Specific examples of the core particle include organic particles, inorganic particles or organic/inorganic composite particles made of plastics, metals, carbon, natural polymers, ceramics (including inorganic solids) and so forth.
Examples of the plastics include polyethylenes, polystyrenes, polycarbonates, polypropylenes, polyamides, phenol resins, epoxy resins, polycarbodiimide resins, polyvinyl chlorides, polyvinylidene fluorides, polyethylene fluorides, polyimides, acrylic resins and so forth.
Examples of the inorganic polymers include glass, quartz, carbon, silica gel, graphite and so forth.
Examples of the metals include metals existing as solids at an ordinary temperature such as gold, platinum, silver, copper, iron, aluminum, magnet, paramagnet and apatite.
Examples of the natural polymers include cellulose, cellulose derivatives, chitin, chitosan, alginic acid and so forth.
Examples of the ceramics include alumina, silica, silicon oxide, aluminum hydroxide, magnesium hydroxide, silicon carbide, silicon nitride, boron carbide and so forth.
One kind of the aforementioned materials alone can be used, or two or more kinds of them can be used in combination as a composite particle.
If the core particle is commercially available, it may be used. Alternatively, the core particle may be produced by any of various known methods. For example, if a desired particle is an organic particle or an organic/inorganic composite particle, the following methods can be used. However, the methods are not particularly limited to these methods.

(i) A method of obtaining particles by pulverizing and classifying a solution resin obtained by usual bulk polymerization or solution polymerization.
(ii) A method of obtaining particles (including spherical particles) by adding dropwise a solution resin obtained by polymerization similar to those mentioned above.
(iii) A method of obtaining particles (including spherical particles) by emulsification or suspension polymerization performed in an aqueous solution.
(iv) A method of obtaining particles by employing the method of (iii) in combination with the seeding method or the like.
(v) A method of obtaining particles (mainly spherical particles) by dispersion polymerization in a non-aqueous solvent or a mixed solvent with water.
(vi) A method of obtaining particles by employing the method of (v) in combination with the seeding method or the like.
(vii) A method of obtaining pellet-like particles using an extruder or the like.

Further, the particles obtained by the aforementioned polymerization may originally have a crosslinked structure, and such particles can also be used for the production according to the present invention.

Preferably, a surface portion, or inside and surface portions of the core particle desirably contain a compound (also referred to as "compound B" hereinafter) having a functional group that can bond to the organic compound A described later by copolymerizing or mixing the compound. For example, when the base particle is produced, the core particle may be modified beforehand, if necessary, for bonding of the core particle and the organic compound A. Further, the organic compound A may also be added to the core particle beforehand. The expression "modification" of the core particle includes both of the case where a functional group is later introduced into a base material from which the core particle is formed, and the case where a base material having a functional group is produced by using a compound originally having a functional group. The compound B will be explained later.

Various known methods can be adopted as the method of incorporating the aforementioned compound B into the core particle. Examples of such methods include, when the core particle is a polymer particle derived from unsaturated monomers, a method of copolymerizing unsaturated monomers having a functional group that can bond to the organic compound A during polymerization of the polymer to produce particles and so forth.

More specific examples include, when the particle to be used as a core is a metal or an inorganic particle such as those of silicon oxide, aluminum hydroxide or magnesium hydroxide, a method of treating the surface of the particle with a silane coupling agent having a functional group that can bond to the organic compound A to form the core particle and so forth.

Further, when the particle to be used as a core is a composite particle comprising organic and inorganic materials (polymer particles containing a magnetic substance etc.), for example, the core particle can also be produced by employing the aforementioned methods in combination depending on the amounts of organic and inorganic material components.

The device of the present invention and the core particle serving as a support therefor may have an irregular shape or spherical shape depending of the use of the device. However, because highly precise devices and particles with uniform surface areas have been required for medical use in recent years, particles having uniform particle diameters or particles of a spherical or substantially spherical shape are preferred.

(2) Organic compound A

The organic compound A is a compound having at least one functional group A1 that can bond to the core particle and one functional group A2 that can chemically bond to the biologically active substance as well as two or more hydrophilic groups. The functional group A1 that can bond to the core particle and the functional group A2 that can bond to the biologically active substance may be identical or different.

Examples of the aforementioned functional groups A1 and A2 include carbodiimide group, ester group, carbonate group, epoxy group, oxazoline group and so forth.

The aforementioned hydrophilic group is not particularly limited so long as it is a functional group that is swollen or dissolved in water. Specific examples thereof include hydroxyl group, ncarboxyl group, ethylene oxide group, propylene oxide group, phosphoric acid group, sulfonic acid group, heterocyclic hydrophilic functional group containing nitrogen and so forth. A molecule of the organic compound A preferably contains two or more, preferably 8 or more, more preferably 12 or more hydrophilic groups. Further, the upper limit number of the hydrophilic groups is usually 60 or less, preferably 60 or less, more preferably 40 or less. The hydrophilic group is preferably a hydrophilic group that makes the organic compound A containing it water-soluble.

Among the aforementioned functional groups, carbodiimide group is preferred, and in particular, a water-soluble carbodiimide compound is preferred. Hereafter, the organic compound A containing carbodiimide group will be described with reference to examples thereof. The organic compound A having carbodiimide group is preferably a compound represented by the following formula.

$$A_x\text{- }(R\text{-}X)_n\text{-}R\text{-}A_y \qquad \text{(I)}$$

$A_x$ and $A_y$ independently represent a segment having a functional group that exhibits hydrophilicity and may be identical to or different from each other. R independently represents an organic group of two or more valences, X independently represents carbodiimide group, epoxy group or oxazoline group, and n is an integer of 2 to 80, preferably 2 to 40.

Examples of the aforementioned organic group of two or more valences include hydrocarbon groups, organic groups containing nitrogen atom or oxygen atom and so forth. Divalent hydrocarbon groups are preferred, and examples thereof include C1 to C12 alkylene groups, C3 to C10 cycloalkylene groups, C4 to C16 alkylene groups having a cyclic or non-cyclic structure, C6 to C16 divalent aromatic rings, C7 to C18 alkylene groups containing an aromatic ring and so forth.

Examples of the organic compound A having carbodiimide group and represented by the aforementioned formula (I) (also referred to simply as "carbodiimide compound" hereinafter) include polycarbodiimides that can be produced by the method disclosed in Japanese Patent Laid-open No. 51-61599, the method of L.M. Alberino et al. (J. Appl. Polym. Sci., 21, 190 (1990)), the method disclosed in Japanese Patent Laid-open No. 2-292316 or so forth. That is, those compounds can be produced from organic polyisocyanate compounds in the presence of a catalyst that promotes carbodiimidation of isocyanates.

Examples of the aforementioned organic polyisocyanate compounds used for the production of polycarbodiimides include 4,4'-dicyclohexylmethane diisocyanate, m-tetramethylxylylene diisocyanate, 2,4-tolylene diisocyanate, 2,6-tolylene diisocyanate, a mixture of 2,4-tolylene diisocyanate and 2,6-tolylene diisocyanate, crude tolylene diisocyanate, crude methylene diphenyl diisocyanate, 4,4',4"-triphenylmethylene triisocyanate, xylene diisocyanate, hexamethylene-1,6-diisocyanate, lysine diisocyanate, hydrogenated methylene diphenyl diisocyanate, m-phenyl diisocyanate, naphthylene-1,5-diisocyanate, 4,4'-biphenylene diisocyanate, 4,4'-diphenylmethane diisocyanate, 3,3'-dimethoxy-4,4'-biphenyl diisocyanate, 3,3'-dimethyldiphenylmethane-4,4'-diisocyanate, isophorone diisocyanate and arbitrary mixtures thereof.

Carbodiimidation of isocyanate groups in the aforementioned polyisocyanate compounds or mixtures thereof causes condensation polymerization. This reaction is usually performed by heating an isocyanate in the presence of a carbodiimidation catalyst. In this reaction, the molecular weight (degree of polymerization) of the product can be controlled by adding a compound having a functional group exhibiting reactivity with an isocyanate group, for example, hydroxyl group, primary or secondary amino group, carboxyl group, thiol group or the like as well as a hydrophilic functional group in the molecule as an end blocking agent at a suitable stage to block the end of the carbodiimide compound. The degree of polymerization can also be controlled by changing concentrations of polyisocyanate compounds or the like and reaction time.

Examples of the aforementioned catalyst that promotes carbodiimidation of organic isocyanates include various substances, and 1-phenyl-2-phospholene-1-oxide, 3-methyl-1-phenyl-2-phospholene-1-oxide, 1-ethyl-2-phospholene-1-oxide, 3-phospholene isomers thereof and so forth are preferred in view of yield and other factors.

The carbodiimide compounds represented by the aforementioned chemical formula (I) usually have an average molecular weight of 200 to 100,000, preferably 500 to 50,000.

As described above, to produce the carbodiimide compound according to the present invention, the aforementioned isocyanate is first heated in the presence of a carbodiimidation catalyst. In this case, the synthesis may be performed with or without a solvent. Further, a solvent may be added during the process of the reaction. In such a case, the solvent can be suitably selected depending on the purpose of use.

Specifically, typical examples of the solvent include ketones such as acetone, methyl ethyl ketone, methyl isobutyl ketone and cyclohexanone; esters such as ethyl acetate, butyl acetate, ethyl propionate and cellosolve acetate; aliphatic or aromatic hydrocarbons such as pentane, 2-methylbutane, n-hexane, cyclohexane, 2-methylpentane, 2,2-dimethylbutane, 2,3-dimethylbutane, heptane, n-octane, isooctane, 2,2,3-trimethylpentane, decane, nonane, cyclopentane, methylcyclopentane, methylcyclohexane, ethylcyclohexane, p-menthane, benzene, toluene, xylene and ethylbenzene; halogenated hydrocarbons such as carbon tetrachloride, trichloroethylene, chlorobenzene and tetrabromoethane; ethers such as ethyl ether, dimethyl ether, trioxane and tetrahydrofuran; acetals such as methylal and diethylacetal; organic compounds containing sulfur or nitrogen such as nitropropene, nitrobenzene, pyridine, dimethylformamide and dimethyl sulfoxide and so forth. The solvent is not particularly limited so long as it does not adversely affect the isocyanate group and the carbodiimide group at the time of the synthesis, and the solvent can be suitably selected depending on the purpose of the polymerization. Further, one kind of these solvents alone can be used, or two or more kinds of them may be used in combination.

Further, if the carbodiimide resin ends are blocked with a hydrophilizing segment described below after completion of the synthesis, water, alcohols such as methanol, ethanol, 1-propanol, 2-propanol, 1-butanol, 2-butanol, isobutyl alcohol, tert-butyl alcohol, 1-pentanol, 2-pentanol, 3-pentanol, 2-methyl-1-butanol, isopentyl alcohol, tert-pentyl alcohol, 1-hexanol, 2-methyl-1-pentanol, 4-methyl-2-pentanol, 2-ethylbutanol, 1-heptanol, 2-heptanol, 3-heptanol, 2-octanol, 2-ethyl-1-hexanol, benzyl alcohol and cyclohexanol; ether alcohols such as methyl cellosolve, ethyl cellosolve, isopropyl cellosolve, butyl cellosolve and diethylene glycol monobutyl ether and so forth can be used as a diluent in addition to the aforementioned solvents. One kind of these alone can be used, or two or more kinds of them may be used in combination. It is preferable to use a relatively low temperature during the dilution, because carbodiimide group is highly reactive.

By changing molecular weight or composition of the organic compound A such as those carbodiimide compounds or the hydrophilizing segment, dispersibility of the base particle can be freely controlled, and degrees of aggregation and dispersion of the device itself can be controlled as required.

The hydrophilizing segment ($A_x$ and Ay in the aforementioned formula) is not particularly limited so long as it is, for example, a segment that has a hydrophilic group and can become water-soluble. Preferred examples thereof include residues of alkylsulfonates having at least one of reactive hydroxyl group such as sodium hydroxyethanesulfonate and sodium hydroxypropanesulfonate, quaternary salts of residues of dialkylaminoalcohols such as 2-dimethylaminoethanol, 2-diethylaminoethanol, 3-dimethylamino-1-propanol, 3-diethylamino-1-propanol, 3-diethylamino-2-propanol, 5-diethylamino-2-propanol and 2-(di-n-butylamino)ethanol, quaternary salts of residues of dialkylaminoalkylamines such as 3-dimethylamino-n-propylamine, 3-diethylamino-n-propylamine and 2-(diethylamino)ethylamine and residues of poly(alkylene oxides) having at least one of reactive hydroxyl group such as poly(ethylene oxide) monomethyl ether, poly(ethylene oxide) monoethyl ether, poly(ethylene oxide/propylene oxide) monomethyl ether and poly(ethylene oxide/propylene oxide) monoethyl ether. One kind of these segments ($A_x$ and Ay) that become hydrophilic alone can be used, or two or more kinds of them may be used in combination, and they can also be used as copolymerized mixed compounds.

Because devices used for medical test, diagnosis or therapeutic treatment, in particular, are often used with water-soluble media including water, dispersibility of the base particle greatly affect the precision of the device.

For the production of the base particle, one kind of the aforementioned organic compounds A alone can be used, or two or more kinds of them may be used in combination. They may also be used as copolymerized mixed compounds.

(3) Base particle

The base particle consists of the aforementioned core particle bonded with the organic compound A by a chemical bond. In the present invention, the "chemical bond" means a bond such as covalent bond, coordinate bond or ionic bond.

As for the production method of the base particle, the base particle can be obtained by, for example, preparing a core particle containing a compound B having a functional group that can react with an organic compound A, adding the organic compound A to the core particle in the presence of a solvent in which the core particle is not dissolved and the organic compound A is dissolved to allow a chemical reaction, without deforming the shape of the particle. In the production, if the core particle and the organic compound A are not chemically bonded in the base particle, impurities or undesired substances are often dissolved or precipitated in the solution in the following processes, or the particles often aggregate. As a result, the obtained device can no longer maintain high precision required as a device for test, diagnosis or therapeutic treatment.

Hereafter, the method of producing the base particle in which a core particle and an organic compound A are bonded will be explained by referring to a case where polymer particles are used as the core particle as an example.

Examples of the polymer particles that can be used for the core particle include, for example, those of styrene polymers, (meth)acrylic polymers, copolymers obtained by addition polymerization of other vinyl polymers, polymers obtained by hydrogen transfer polymerization, polymers obtained by polycondensation, polymers obtained by addition condensation and so forth.

Typical examples of copolymerizable raw material monomers as the main component include (i) styrenes such as styrene, o-methylstyrene, m-methylstyrene, p-methylstyrene, α-methylstyrene, p-ethylstyrene, 2,4-dimethylstyrene, p-n-butylstyrene, p-tert-butylstyrene, p-n-hexylstyrene, p-n-octylstyrene, p-n-nonylstyrene, p-n-decylstyrene, p-n-dodecylstyrene, p-methoxystyrene, p-phenylstyrene, p-chlorostyrene and 3,4-dichlorostyrene, (ii) (meth)acrylic acid esters such as methyl acrylate, ethyl acrylate, n-butyl acrylate, isobutyl acrylate, propyl acrylate, hexyl acrylate, 2-ethylhexyl acrylate, n-octyl acrylate, dodecyl acrylate, lauryl acrylate, stearyl acrylate, 2-chloroethyl acrylate, phenyl acrylate, methyl α-chloroacrylate, methyl methacrylate, ethyl methacrylate, n-butyl methacrylate, isobutyl methacrylate, propyl methacrylate, hexyl methacrylate, 2-ethylhexyl methacrylate, n-octyl methacrylate, dodecyl methacrylate, lauryl methacrylate and stearyl methacrylate, (iii) vinyl esters such as vinyl acetate, vinyl propionate, vinyl benzoate and vinyl butyrate, (iv) (meth)acrylic acid derivatives such as acrylonitriles and methacrylonitriles, (v) vinyl ethers such as vinyl methyl ether, vinyl ethyl ether and vinyl isobutyl ether, (vi) vinyl ketones such as vinyl methyl ketone, vinyl hexyl ketone and methyl isopropenyl ketone, (vii) N-vinyl compounds such as N-vinylpyrrole, N-vinylcarbazole, N-vinylindole and N-vinylpyrrolidone, (viii) vinyl fluoride, vinylidene fluoride, tetrafluoroethylene, hexafluoropropylene, (meth)acrylic esters having a fluorinated alkyl group such as trifluoroethyl acrylate and tetrafluoropropyl acrylate and so forth. One kind of these alone can be used, or two or more kinds of them may be used in combination.

It is sufficient that the core particle in the base particle according to the present invention should contain a compound B containing a functional group B1 that can bond to an organic compound A in a surface portion or inside and surface portions thereof. Examples of the aforementioned functional group B1 include, for example, compounds having groups containing a carbon-carbon unsaturated bond (double bond, triple bond), α,β-unsaturated carbonyl group, epoxy group, isocyanate group, carboxyl group, hydroxyl group, amido group, thiol group, cyano group, amino group, chloromethyl group, glycidyl ether group, ester group, formyl group, nitrile group, nitroso group, carbodiimide group, oxazoline group or the like. One kind of these alone can be used, or two or more kinds of them may be used in combination. Carboxyl group, hydroxyl group, primary or secondary amino group or thiol group are preferred.

Further, specific examples of the compound B include radically polymerized monomers and compounds containing carboxyl group. Typical examples thereof include various unsaturated mono- or dicarboxylic acids or unsaturated dibasic acids such as acrylic acid, methacrylic acid, crotonic acid, itaconic acid, maleic acid, fumaric acid, monobutyl itaconate and monobutyl maleate and so forth. One kind of these compounds alone can be used, or two or more kinds of them may be used in combination.

Examples of the compound B further include radically polymerized monomers and compounds having hydroxyl group. Typical examples thereof include (meth)acrylic monomers such as 2-hydroxyethyl (meth)acrylate, 2-hydroxypropyl (meth)acrylate, 3-hydroxypropyl (meth)acrylate and 4-hydroxybutyl (meth)acrylate, polyalkylene glycol (meth)acrylate compounds such as polyethylene glycol mono(meth)acrylate and polypropylene glycol mono(meth)acrylate, various hydroxyalkyl vinyl ethers such as hydroxyethyl vinyl ether and hydroxybutyl vinyl ether, various allyl compounds such as allyl alcohol and 2-hydroxyethyl allyl ether and so forth. One kind of these compounds alone can be used, or two or more kinds of them may be used in combination.

Examples of the compound B further include polymers containing hydroxyl group. Typical examples thereof include thermoplastic resins containing hydroxyl group such as completely or partially saponified resins of polyvinyl alcohol (PVA) and saponified resins of polymers containing acetic acid ester comprising a copolymer of vinyl acetate and other vinyl monomers. One kind of these compounds alone may be used, or two or more kinds of them may be used in combination.

Examples of the compound B further include radically polymerized monomers and compounds containing amino group. Typical examples thereof include, specifically, derivatives of alkyl esters of acrylic or methacrylic acids such as aminoethyl acrylate, N-propylaminoethyl acrylate, N-ethylaminopropyl methacrylate, N-phenylaminoethyl methacrylate and N-cyclohexylaminoethyl methacrylate; allylamine and allyl amine derivatives such as N-methylallylamine; styrene derivatives such as p-aminostyrene; triazine derivatives such as 2-vinyl-4,6-diamino-S-triazine and so forth, and compounds containing primary or secondary amino group are preferred. One kind of these compounds alone may be used, or two or more kinds of them may be used in combination.

Examples of the compound B further include radically polymerized monomers and compounds containing thiol (mercapto) group. Typical examples thereof include, specifically, monomers or compounds containing mercapto (thiol) group and having an unsaturated double bond such as 2-propene-1-thiol, 3-butene-1-thiol, 4-pentene-1-thiol, 2-mercaptoethyl (meth)acrylate, 2-mercapto-1-carboxyethyl (meth)acrylate, N-(2-mercaptoethyl)acrylamide, N-(2-mercapto-1-carboxyethyl)acrylamide, N-(2-mercaptoethyl)methacrylamide, N-(4-mercaptophenyl)acrylamide, N-(7-mercaptonaphthyl)acrylamide and mono-2-mercaptoethylamide maleate and so forth. One kind of these compounds alone may be used, or two or more kinds of them may be used in combination. Examples further include thermoplastic resins having thiol (mercapto) group such as modified polyvinyl alcohols having thiol (mercapto) group and so forth.

Further, when a composite group of carboxyl group, hydroxyl group, amino group, thiol (mercapto) group etc. is desired to be introduced into a copolymer that forms the core particle, a polyfunctional copolymer can be produced by using two or more kinds of monomers containing any of the aforementioned various reactive groups in combination.

A polyfunctional base particle can be produced by controlling the amounts of the aforementioned functional groups, amount of the organic compound A to be added, reaction temperature and other conditions during the reaction of the core particle and the organic compound A.

As a polymerization initiator used in the polymerization of radically polymerizable monomers for the production of the core particle, known radical polymerization initiators can be used. Typical examples thereof include, specifically, peroxides such as benzoyl peroxide, cumene hydroperoxide, t-butyl hydroperoxide, sodium persulfate and ammonium persulfate, azo compounds such as azobisisobutyronitrile, azobismethylbutyronitrile and azobisvaleronitrile and so forth. One kind of these compounds alone may be used, or two or more kinds of them may be used in combination.

For the production of the core particle, various synthesis and polymerization methods such as those mentioned above are used. Examples include not only synthesis without solvent such as bulk polymerization but also synthesis in a solvent such as solution polymerization. Specific examples of the polymerization solvent include water, alcohols such as methanol, ethanol, 1-propanol, 2-propanol, 1-butanol, 2-butanol, isobutyl alcohol, tert-butyl alcohol, 1-pentanol, 2-pentanol, 3-pentanol, 2-methyl-1-butanol, isopentyl alcohol, tert-pentyl alcohol, 1-hexanol, 2-methyl-1-pentanol, 4-methyl-2-pentanol, 2-ethylbutanol, 1-heptanol, 2-heptanol, 3-heptanol, 2-octanol, 2-ethyl-1-hexanol, benzyl alcohol and cyclohexanol; ether alcohols such as methyl cellosolve, ethyl cellosolve, isopropyl cellosolve, butyl cellosolve and diethylene glycol monobutyl ether; ketones such as acetone, methyl ethyl ketone, methyl isobutyl ketone and cyclohexanone; esters such as ethyl acetate, butyl acetate, ethyl propionate and cellosolve acetate; aliphatic or aromatic hydrocarbons such as pentane, 2-methylbutane, n-hexane, cyclohexane, 2-methylpentane, 2,2-dimethylbutane, 2,3-dimethylbutane, heptane, n-octane, isooctane, 2,2,3-trimethylpentane, decane, nonane, cyclopentane, methylcyclopentane, methylcyclohexane, ethylcyclohexane, p-menthane, dicyclohexyl, benzene, toluene, xylene and ethylbenzene; halogenated hydrocarbons such as carbon tetrachloride, trichloroethylene, chlorobenzene and tetrabromoethane; ethers such as ethyl ether, dimethyl ether, trioxane and tetrahydrofuran; acetals such as methylal and diethylacetal; fatty acids such as formic acid, acetic acid and propionic acid; organic compounds containing sulfur or nitrogen such as nitropropene, nitrobenzene, dimethylamine, monoethanolamine, pyridine, dimethylformamide, dimethyl sulfoxide and acetonitrile and so forth. The polymerization solvent is not particularly limited and may be suitably selected depending on the purpose of use of the polymerization method. One kind of these solvents alone may be used, or two or more kinds of them may be used in combination.

Further, in the production of the core particle, a dispersing agent, stabilizer, emulsifier (or surfactant), antioxidant, catalyst (or reaction accelerator) and so forth may be suitably used depending on the polymerization method that can be used.

Typical examples of the dispersing agent and the stabilizer include, specifically, various hydrophobic or hydrophilic dispersing agents and stabilizers, for example, polystyrene derivatives such as polyhydroxystyrene, polystyrenesulfonic acid, vinylphenol/(meth)acrylic acid ester copolymer, styrene/(meth)acrylic acid ester copolymers and styrene/vinylphenol/(meth)acrylic acid ester copolymers; poly(meth)acrylic acid derivatives such as poly(meth)acrylic acid, poly(meth)acrylamide, polyacrylonitrile, polyethyl (meth)acrylate and polybutyl (meth)acrylate; polyvinyl alkyl ether derivatives such as polymethyl vinyl ether, polyethyl vinyl ether, polybutyl vinyl ether and polyisobutyl vinyl ether; cellulose derivatives such as cellulose, methylcellulose, cellulose acetate, cellulose nitrate, hydroxymethylcellulose, hydroxyethylcellulose, hydroxypropylcellulose and carboxymethylcellulose; polyvinyl acetate derivatives such as polyvinyl alcohol, polyvinyl butyral, polyvinyl formal and polyvinyl acetate; nitrogen-containing polymer derivatives such as polyvinylpyridine, polyvinylpyrrolidone, polyethylenimine and poly-2-methyl-2-oxazoline; poly(halogenated vinyl derivatives) such as polyvinyl chloride and polyvinylidene chloride; polysiloxane derivatives such as polydimethylsiloxane and so forth. One kind of these compounds alone may be used, or two or more kinds of them may be used in combination.

Examples of the emulsifier (surfactant) include anionic emulsifiers, for example, alkylsulfuric ester salts such as sodium laurylsulfate, alkylbenzenesulfonic acid salts such as sodium dodecylbenzenesulfonate, alkylnaphthalenesulfonic acid salts, fatty acid salts, alkylphosphoric acid salts and alkylsulfosuccinic acid salts; cationic emulsifiers such as alkylamine salts, quaternary ammonium salts, alkylbetaines and amine oxides; nonionic emulsifiers such as polyoxyethylene alkyl ethers, polyoxyethylene alkyl ethers, polyoxyethylene alkylallyl ethers, polyoxyethylene alkylphenyl ethers, sorbitan fatty acid esters, glycerin fatty acid esters and polyoxyethylene fatty acid esters and so forth. One kind of these compounds alone may be used, or two or more kinds of them may be used in combination.

Further, in the production of the core particle, a small amount of a crosslinking agent may be added depending on the purpose of use. Typical examples thereof include, specifically, aromatic divinyl compounds such as divinylbenzene and divinylnaphthalene; and other compounds such as ethylene glycol diacrylate, ethylene glycol dimethacrylate, triethylene glycol dimethacrylate, tetraethylene glycol dimethacrylate, 1,3-butylene glycol dimethacrylate, trimethylolpropane triacrylate, trimethylolpropane trimethacrylate, 1,4-butanediol diacrylate, neopentyl glycol diacrylate, 1,6-hexanediol diacrylate, pentaerythritol triacrylate, pentaerythritol tetraacrylate, pentaerythritol dimethacrylate, pentaerythritol tetramethacrylate, glycerol acroxydimethacrylate, N,N-divinylaniline, divinyl ether, divinyl sulfide and divinyl sulfone. One kind of these compounds alone may be used, or two or more kinds of them may be used in combination.

When the core particle is a thermoplastic particle containing organic substances, only the particle surface (surface layer portion) or inside and surface portions may be crosslinked.

Examples of the antioxidant include phenol antioxidants, sulfur antioxidants, phosphorus antioxidants, amine antioxidants, hydroquinone antioxidants, hydroxylamine antioxidants and so forth.

Further, the catalyst (reaction accelerator) is not particularly limited so long it accelerates the reaction, and known catalysts may be used. The catalyst may be suitably selected so that physical properties of the particle should not be adversely affected, and a suitable amount thereof can be added. For example, when at least one of the functional group of the core particle and the functional group of the organic compound A contains epoxy group, a catalyst selected from, specifically, tertiary amines such as benzyldimethylamine, triethylamine, tributylamine, pyridine and triphenylamine; quaternary ammonium compounds such as triethylbenzylammonium chloride and tetramethylammonium chloride; phosphines such as triphenylphosphine and tricyclophosphine; phosphonium compounds such as benzyltrimethylphosphonium chloride; imidazole compounds such as 2-methylimidazole and 2-methyl-4-ethylimidazole; alkaline metal hydroxides such as potassium hydroxide, sodium hydroxide and lithium hydroxide; alkaline metal carbonates such as sodium carbonate and lithium carbonate; alkaline metal salts of organic acids; halogenides exhibiting properties of Lewis acid such as boron trichloride, boron trifluoride, tin tetrachloride and titanium tetrachloride and complex salts thereof and so forth can be added. One kind of these compounds alone may be used, or two or more kinds of them may be used in combination.

When the aforementioned core particle is a particle containing organic substances, the weight average molecular weight is about 1000 to 3,000,000. When the core particle is a spherical particle, the weight average molecular weight is about 3000 to 1,000,000.

The amount of the core particle having functional groups with which the organic compound A can react, which is used for the production of the base particle, preferably corresponds to, when the core particle is a polymer microparticle, 30 to 2000 equivalents, more preferably 50 to 1000 equivalents, further preferably 80 to 900 equivalents, particularly preferably 100 to 500 equivalents, with respect to the content of the functional group. If the amount exceeds 2000 equivalents, the bonding to the core particle requires considerable time because the amount of the functional groups becomes too small, and thus such an amount may not be preferred. On the other hand, if the amount is less than 30 equivalents, the bonding density becomes too high, and functional groups that can bond to a biologically active substance may not be left on the surface and the surface layer portion of the base particle. However, if there is extra time or a minimal amount of the functional groups is required, the amount may not be within the range defined above. That is, the amount of the core particle may be more than 2000 equivalents per functional group or less than 30 equivalents per functional group.

This also applies to the cases where the core particle is an inorganic particle, organic/inorganic composite particle or the like.

The aforementioned term "equivalent" means a certain amount assigned to each compound on the basis of quantitative relations of substances in a chemical reaction. For example, the equivalent of the core particle of the present invention means the chemical formula weight of the core particle per mole of the functional group that can react with the organic compound A.

The amount of the organic compound A required to produce the base particle is 50 to 1500 equivalents, preferably 80 to 1000 equivalents, more preferably 100 to 800 equivalents, particularly preferably 200 to 700 equivalents, with respect to the functional group. If the amount exceeds 1500 equivalents, the bonding to the core particle requires considerable time because the amount of the functional group becomes too small, and thus such an amount may not be preferred. On the other hand, if the amount is less than 50 equivalents, a lot of functional groups that can bond to a biologically active substance are remained, and they may provide bad influences. However, if there is extra time or a minimal amount of the functional groups is required, the amount may not be within the range defined above. That is, the amount may be more than 1500 equivalents per functional group.

In the production of the base particle, although the amount of the organic compound A to be added to the core particle depends on the required amount of residual organic compounds after curing or bonding, the organic compound A may be added in an amount of about 0.1 to 20, preferably 0.5 to 8, more preferably 1 to 5, in terms of the equivalent ratio with respect to the functional group of the core particle. This is also applicable to the cases where the particle to be used as the core is a core particle made of an organic/inorganic composite particle or an inorganic particle. Further, the addition amount of the organic compound A may exceed 20 in terms of the equivalent ratio. However, such an amount results in a large amount of residual organic compounds in the medium and thus is not preferred in view of cost. Further, if the addition amount is less than 0.1 in terms of the equivalent ratio, the functional group that can bond to a biologically active substance may not be remained. However, if there is extra time or a minimal amount of the functional groups is required, the amount may not be within the range defined above.

The average particle diameter of the base particle is preferably 0.01 to 100 μm, more preferably 0.05 to 50 μm, further preferably 0.08 to 30 μm, particularly preferably 0.1 to 10 μm. If the average particle diameter exceeds 100 μm, the precipitation rate of the particle increases, and this is not preferable as a device for biological or medical use. On the other hand, if the average particle diameter is less than 0.01 μm, the degree of aggregation becomes high because the particle diameter is too small, and monodispersed particles may not be obtained. It is preferable that diameters of 80% or more, preferably 90% or more, more preferably 95% or more, of the base particles satisfy the aforementioned range.

Although the reaction temperature of the reaction for obtaining the base particle depends on the type of the solvent, it is preferably within the range of 10 to 200°C, more preferably 15 to 150°C, further preferably 20 to 130°C.

Further, the reaction time may be time required to almost complete the bonding reaction of the core particle and the functional group of the organic compound A. Although it largely depends on the type and amount of the organic compound used, the type of the functional group in the particle, viscosity and concentration of the solution and so forth, it is, for example, about 1 to 24 hours, preferably 2 to 15 hours, at 50°C. The base particle can be obtained even if the aforementioned factors are changed to extend the reaction time (longer than 24 hours). However, prolonged time may not be preferred in view of production method. Preferred reaction time can be easily determined by performing the reaction using various reaction times in preliminary experiments.

The solvent in which the core particle is not dissolved and the organic compound A is dissolved is at least one kind of solvent selected from water and organic solvents, and may be suitably selected considering the type and amount of the organic compound used, type of the resin to be used as a component of the base particle, the type of the contained functional group, the purpose of use and so forth.

Specific examples of the solvent include water, alcohols such as methanol, ethanol, 1-propanol, 2-propanol, 1-butanol, 2-butanol, isobutyl alcohol, tert-butyl alcohols, 1-pentanol, 2-pentanol, 3-pentanol, 2-methyl-1-butanol, isopentyl alcohol, tert-pentyl alcohols, 1-hexanol, 2-methyl-1-pentanol, 4-methyl-2-pentanol, 2-ethylbutanol, 1-heptanol, 2-heptanol, 3-heptanol, 2-octanol, 2-ethyl-1-hexanol, benzyl alcohol and cyclohexanol; ether alcohols such as methyl cellosolve, ethyl cellosolve, isopropyl cellosolve, butyl cellosolve and diethylene glycol monobutyl ether; ketones such as acetone, methyl ethyl ketone, methyl isobutyl ketone and cyclohexanone; esters such as ethyl acetate, butyl acetate, ethyl propionate and cellosolve acetate; aliphatic or aromatic hydrocarbons such as pentane, 2-methylbutane, n-hexane, cyclohexane, 2-methylpentane, 2,2-dimethylbutane, 2,3-dimethylbutane, heptane, n-octane, isooctane, 2,2,3-trimethylpentane, decane, nonane, cyclopentane, methylcyclopentane, methylcyclohexane, ethylcyclohexane, p-menthane, dicyclohexyl, benzene, toluene, xylene and ethylbenzene; halogenated hydrocarbons such as carbon tetrachloride, trichloroethylene, chlorobenzene and tetrabromoethane; ethers such as ethyl ether, dimethyl ether, trioxane and tetrahydrofuran; acetals such as methylal and diethylacetal; fatty acids such as formic acid, acetic acid and propionic acid; organic compounds containing sulfur or nitrogen such as nitropropene, nitrobenzene, dimethylamine, monoethanolamine, pyridine, dimethylformamide, dimethyl sulfoxide and acetonitrile and so forth. Preferred examples include water-soluble or hydrophilic media including water, lower alcohols such as methanol and ethanol, ether alcohols such as methyl cellosolve and ethyl cellosolve, mixtures of water and a lower alcohol and mixtures of water and an ether alcohol, toluene, dimethylformamide (DMF), tetrahydrofuran (THF), methyl ethyl ketone (MEK), methyl isobutyl ketone (MIBK), acetone, N-methyl-2-pyrrolidone (NMP), dichloromethane, tetrachloroethylene and so forth. Further preferred are water-soluble or hydrophilic media including water, lower alcohols such as methanol and ethanol, mixtures of water and a lower alcohol such as methanol and ethanol, mixtures of water and a lower alcohol such as methanol and ethanol, and mixtures of water and an ether alcohol. These solvents are not particularly limited, and a solvent suitable for the purpose of use may be selected as required. One kind of these solvents alone may be used, or two or more kinds of them may be used in combination.

In the production of the base particle, the aforementioned dispersing agents, antioxidants, stabilizers, emulsifiers (surfactants), catalysts (reaction accelerators) and so forth can also be suitably selected and added as required.

In the production of the base particle, the solution concentration used for the reaction of the core particle and the organic compound A is 1 to 60% by weight, preferably 5 to 40% by weight, more preferably 10 to 30% by weight, as calculated according to the following equation.

$$\text{Solution concentration (\% by weight)} = [(\text{Total solution} - \text{Solvent})/\text{Total solution}] \times 100$$

If the aforementioned solution concentration exceeds 80% by weight, the amount of the core particle or the organic compound A becomes excessive, therefore balance in the solution is deteriorated, and it becomes difficult to obtain stable monodispersed particles. Therefore, such a concentration is not preferred. Further, if the aforementioned solution concentration is less than 1% by weight, although the base particle can be produced, synthesis needs to be performed over a long period of time to obtain objective particles. In view of production, it is not desirable to be required a long period of time.

As described above, as for the shape of the base particle, the particles preferably have uniform particle diameters and have a spherical or substantially spherical shape. In the present invention, the "spherical or substantially spherical" shape is defined as a shape satisfying the condition of "$1 \leq$ Major axis/Minor axis $\leq 1.2$" in a two-dimensional projection drawing of the particle. The major axis and the minor axis can be measured as follows, for example. Particles are photographed by using a scanning electron microscope (hereinafter referred to as "SEM", e.g., Hitachi S-2150) at a measurable magnification (x 100 to 10,000), and the diameter of one particle is randomly measured 15 times to measure the major axis and the minor axis. This procedure is randomly repeated (for example, n = 100) to measure them.

Further, the average particle diameter can be obtained by photographing the particles using SEM with a measurable magnification (x 100 to 10,000), randomly measuring diameters of particles (for example, $n_1$ = 500 particles) and calculating the average of the diameters as the average particle diameter.

Further, from the measurement results of the aforementioned particle diameters of the core particles and the base particles, the CV (coefficient of variation) value for the particle diameter distribution as defined by the following equation can be obtained to confirm distribution accuracy for each of the core particle, the base particle and the device.

CV (%) = (Standard deviation of particle [device] diameter/Average particle [device] diameter) $\times$ 100

Further, dispersibility of the base particle and the device of the present invention can be represented by the CV ratios defined as the following equations. It is preferred that, among these CV ratios, at least one of the $CV_b$ ratio and the $CV_c$ ratio is 0.6 to 3.0, preferably 0.8 to 1.5, more preferably 0.9 to 1.1. Further, it is more preferred that both of the aforementioned $CV_b$ ratio and $CV_c$ ratio are within the aforementioned range. Further, it is particularly preferred that the $CV_a$ ratio is also within the aforementioned ranges in addition to the $CV_b$ ratio and the $CV_c$ ratio.

$$CV_a \text{ ratio} = CV_1/CV_2$$

$$CV_b \text{ ratio} = CV_1/CV_3$$

$$CV_c \text{ ratio} = CV_2/CV_3$$

$CV_1$ = (Standard deviation of core particle diameter/Average core particle diameter) × 100
$CV_2$ = (Standard deviation of base particle diameter/Average base particle diameter) × 100
$CV_3$ = (Standard deviation of device diameter/Average device particle diameter) × 100

(4) Biologically active substance

The biologically active substance is for capturing a second biologically active substance that specifically bonds to the substance. Examples of the second biologically active substance to be detected include nucleic acids, proteins (including peptides), saccharides and so forth. Among them, nucleic acids are preferred. Further, when the device of the present invention is used for therapeutic treatment, the biologically active substance functions as an active ingredient of an agent for therapeutic treatment.

When a nucleic acid is used as the biologically active substance, it may not be particularly different from nucleic acids used for usual hybridization of nucleic acids using nucleic acids immobilized on a solid phase, and it is not particularly limited so long as it is a nucleic acid that can hybridize. Examples include, for example, naturally occurring and synthesized DNAs (including oligonucleotides) and RNAs (including oligonucleotides). Further, the nucleic acid may be single-stranded or double-stranded. The chain length of the nucleic acid is not particularly limited so long as it allows hybridization. However, it is usually about 5 to 50,000 nucleotides, preferably 20 to 10,000 nucleotides. Further, the nucleic acid may have a polymer containing a group that becomes reactive with an ultraviolet ray such as thymine at the 5' or 3' end.

Hereafter, a method of bonding a nucleic acid as the biologically active substance to the base particle will be exemplified. When other substances are used, the solvent, reaction conditions and so forth can be suitably selected depending on the type of the functional group A2 that can covalently bond to the biologically active substance in the organic compound A, so that a reaction of forming a covalent bond between the biologically active substance and the functional group A should occur.

The solvent for dissolving a nucleic acid is not particularly limited either, and examples thereof include distilled water and buffers usually used for preparation of a nucleic acid solution, for example, Tris buffers such as TE buffer (10 mM Tris/hydrochloric acid, pH 8.0/1 mM EDTA), aqueous solutions containing sodium chloride, aqueous solutions containing a carboxylate (sodium citrate, ammonium citrate, sodium acetate etc.), aqueous solutions containing a sulfonate (sodium dodecylsulfate, ammonium dodecylsulfate etc.), aqueous solutions containing a phosphonate (sodium phosphate, ammonium phosphate etc.) and so forth. Commercially available solvents such as Micro Spotting Solution (TeleChem International, Inc.) etc. can also be mentioned. Further, although the concentration of the nucleic acid solution is not particularly limited either, the concentration is usually 1 mmol/ml to 1 fmol/ml, preferably 100 pmol/ml to 100 fmol/ml.

Examples of the method of bringing a nucleic acid solution into contact with the base particle include a method of adding a nucleic acid solution dropwise onto base particles using a pipette, a method of using a commercially available spotter, a method of suspending base particles in a nucleic acid solution and so forth. Although the amount of the nucleic acid solution is not particularly limited, it is preferably 10 nl to 10 ml. One kind or two or more kinds of nucleic acid solutions can be used. As a positive control for confirming immobilization of the nucleic acid on the base particle, a labeled nucleic acid may be brought into contact with the base particle.

In a preferred embodiment of the present invention, a nucleic acid solution is brought into contact with base particles and irradiated with ultraviolet ray. Further, after the aforementioned nucleic acid solution is brought into contact, the base particles can be dried before ultraviolet ray irradiation. The aforementioned nucleic acid solution may be dried spontaneously or by heating. The temperature for the heating is usually 30 to 100°C, preferably 35 to 45°C.

Subsequently, the base particles are irradiated with an ultraviolet ray. Specifically, the ultraviolet ray may have a broad waveform including a wavelength of 280 nm. The irradiation dose is usually 100 $mJ/cm^2$ or more, preferably 200 $mJ/cm^2$ or more, as a cumulative irradiation dose. Further, a nucleic acid having a photoreactive group introduced into an arbitrary part of the nucleic acid may also be used.

The device of the present invention is produced by immobilizing a nucleic acid onto a base particle as described above. The device obtained by the present invention can be used for, for example, analysis of nucleic acids by hybridization. Because a nucleic acid immobilized on base particles by the method of the present invention exhibits superior dispersibility, more favorable detection sensitivity and reproducibility can be obtained compared with conventional methods. Hybridization and detection thereof can be performed in the same manner as usual hybridization using nucleic acids immobilized on a solid phase.

Further, when a protein is used as the biologically active substance, any of proteins such as antibodies, antigens, enzymes and hormones can be used as in usual solid phase immunological reagents.

<2> Utilization of device of the present invention

In a preferred embodiment, the device of the present invention is used for detection or measurement of a second biologically active substance that specifically bonds to a biologically active substance on the device. In this embodiment, the device of the present invention can also detect or measure a substance that inhibits the binding of the biologically active substance on the device and the second biological substance.

Examples of the aforementioned biologically active substance include nucleic acids, proteins (including peptides), saccharides and so forth. Examples of nucleic acids include DNAs and RNAs, and examples of proteins include antigens, antibodies, enzymes and so forth. The following explanation will be made for a nucleic acid as an example of the biologically active substance. However, except that a hybrid is formed as a detection method unique to nucleic acids, methods and conditions usually used for detection can also be adopted for other substances.

The device of the present invention can be used for detection or purification or as a template for PCR in methods for detecting a nucleic acid by hybridization using a nucleic acid labeled with a labeling substance. That is, a particle comprising a base particle on which a nucleic acid is immobilized (hereinafter, referred to as "device") can be used as a probe for hybridization.

A nucleic acid to be measured can be detected by hybridizing a probe with the nucleic acid to be measured to form a nucleic acid/nucleic acid hybrid, removing free probes from the system and detecting the labeling substance contained in the hybrid. Further, an objective nucleic acid can also be purified in a similar manner. Alternatively, a nucleic acid captured by the nucleic acid immobilized on the device can be used as a template for PCR.

In the present invention, the base particle can be directly detected by measuring fluorescence intensity or the like using a fluorescence spectrophotometer, fluorescence spectrophotometer for a 96-well microtiter plate, fluorescence microscope or the like.

Hybridization using the device of the present invention is not particularly different from usual hybridization of nucleic acids.

Although a nucleic acid used as a sample is preferably labeled by labeling a polynucleotide or oligonucleotide using a method usually used for labeling of a nucleic acid, a nucleic acid can also be labeled by incorporating a labeled nucleotide into a polynucleotide or oligonucleotide using a polymerase reaction.

The device of the present invention shows favorable dispersion stability in an aqueous medium, because the biologically active substance bonds to the base particle via an organic compound having two or more hydrophilic groups. For example, when detection of SNP (Single nucleotide polymorphism) associated with a disease or gene expression analysis is performed by hybridization, conventional devices aggregate with one another in a hybridization solution, and hence devices become unable to keep an appropriate distance between them. As a result, hybridization is easily inhibited by steric hindrance of devices or genes. On the other hand, because of the favorable dispersibility of the devices of the present invention in an aqueous medium, the aforementioned inhibition of hybridization hardly occurs. Further, due to this superior dispersibility in the aqueous solution, detection of specimen using fluorescence, radioisotope or the like can be performed with good reproducibility. As a result, SNP can be detected or gene expression analysis can be performed with high efficiency and sensitivity. In particular, when a biological sample is detected, it is often the case that only a minimal amount of specimen can be collected. Accordingly, establishment of a technique for detecting a small amount of a substance with high efficiency and sensitivity is being required. Because a detection method using the device of the present invention can detect such a minimal amount of specimen with good reproducibility, it would be an effective detection technique.

In the present invention, an aqueous medium means any of water, buffers such as TE buffer, SSC buffer, phosphate buffer, acetate buffer, borate buffer, Tris-HCl buffer, UniHybri™ (Telechem International), ExpressHyb™ Hybridization Solution (Clontech) and SlideHyb™ Survey Kit (Ambion), the aforementioned aqueous media mixed with organic solvents such as DMSO and DMF, the aforementioned aqueous media mixed with surfactants such as SDS (sodium dodecylsulfate), the aforementioned aqueous media mixed with various reagents including onium salts such as tetramethylammonium salt, formamide etc., which can change Tm of a nucleic acid to be hybridized and so forth.

Further, for example, the device of the present invention can be suitably used in detection of SNP using LUMINEX System (Hitachi Software Engineering Co., Ltd.), RT-PCR using GeneAmp 2400 (Perkin Elmer) or TP3000 (TAKARA), isolation of specimen using Te-MagS MBS (Tecan), mRNA Isolation Kit (Roche Diagnostics Corporation) or automatic plasmid extraction apparatus (TAKARA) and so forth.

In another embodiment, the device of the present invention is used for treatment of diseases. In this embodiment, a biologically active substance functions as an active ingredient of a therapeutic agent.

## Examples

The present invention will be explained more specifically with reference to the following examples. However, the scope of the present invention is not limited to these examples. In the following examples, "part" means "weight part", and "water" means "distilled water" unless otherwise indicated.

Example 1: Production of core particles

<1> Production Example 1 of Core Particle

A mixture comprising the following components was charged into a 500-ml flask in a batch, and dissolved oxygen was replaced with nitrogen. Then, the mixture was heated at 68°C for about 15 hours on an oil bath with stirring using a stirrer under a nitrogen flow to obtain a styrene/methacrylic acid copolymer particle solution.

| | |
|---|---|
| Styrene | 48.2 parts |
| Methacrylic acid | 20.6 parts |
| Methanol | 179.8 parts |
| Ethanol | 29.9 parts |
| Water | 59.8 parts |
| Azobis-2-methylbutyronitrile (ABNE) | 3.0 parts |
| Styrene/methacrylic copolymer resin solution | 75.0 parts |

(Styrene:2-hydroxyethyl methacrylate = 2:8, 40% by weight solution in methanol)

Subsequently, a part of this particle solution was repeatedly washed with a mixture of water and methanol (3:7) and filtered 3 to 5 times or so by using suction filtration equipment, and then vacuum-dried to obtain Core Particles 1. When the shapes of the particles were examined by using SEM (S-2150, Hitachi, Ltd.), spherical particles were observed. The particle diameter was measured, and the average particle diameter was found to be 1.42 μm.

<2> Production Example 2 of Core Particle

A mixture comprising the following components was charged into a 500-ml flask in a batch, and dissolved oxygen was replaced with nitrogen. Then, the mixture was heated at 70°C for about 15 hours on an oil bath with stirring by using a stirrer under a nitrogen flow to obtain styrene/methacrylic acid copolymer particle solution.

| | |
|---|---|
| Styrene | 34.4 parts |
| Methacrylic acid | 8.6 parts |
| Methanol | 208.0 parts |
| Water | 52.0 parts |
| Azobis-2-methylbutyronitrile (ABNE) | 1.0 part |
| Polyvinylpyrrolidone (K-90) | 15.0 parts |

Subsequently, a part of this particle solution was repeatedly washed with a mixture of water and methanol (3:7) and filtered 3 to 5 times or so by using suction filtration equipment, and then vacuum-dried to obtain Core Particles 2. When the shapes of the particles were examined by using SEM (S-2150, Hitachi, Ltd.), spherical particles were observed. The particle diameter was measured, and the average particle diameter was found to be 0.78 μm.

The results of the above production of core particles are summarized in Table 1.

Table 1

| | Reactive group contained in particles | Equivalent of functional group in polymer particles | Raw material used |
|---|---|---|---|
| Core Particle 1 | Carboxyl group | 287/COOH | Styrene, methacrylic acid |
| Core Particle 2 | Carboxyl group | 430/COOH | Styrene, methacrylic acid |

Example 2: Synthesis of Organic compound A

<1> Synthesis Example 1 of Polycarbodiimide Compound

In an amount of 500 g of 2,6-tolylene diisocyanate (TDI) and 367.8 g of polyoxyethylene monomethyl ether having a polymerization degree m of 8 were initially reacted at 50°C for 1 hour, then added with 5 g of a carbodiimidation catalyst and reacted at 85°C for 6 hours to obtain a polycarbodiimide compound (polymerization degree = 5) having blocked ends. This compound was gradually added with 508.3 g of distilled water to obtain a solution of Polycarbodiimide Compound 1 (resin concentration: 60% by weight). The carbodiimide equivalent was 318/NCN.

<2> Synthesis Example 2 of Polycarbodiimide Compound

In an amount of 500 g of tetramethylxylylene diisocyanate (TMXDI) and 10 g of a carbodiimidation catalyst were reacted at 180°C for 10 hours to obtain poly-m-tetramethylxylylenecarbodiimide (polymerization degree = 3) having an isocyanate end. This compound was added with 393.5 g of polyoxyethylene monomethyl ether having a polymerization degree m of 8 and reacted at 140°C for 6 hours to obtain Polycarbodiimide Compound 2 having blocked ends. This compound was gradually added with 550.6 g of distilled water to obtain a solution of Polycarbodiimide Compound 2 (resin concentration: 60% by weight). The carbodiimide equivalent was 537/NCN.

<3> Synthesis Example 3 of Polycarbodiimide Compound

In an amount of 500 g of 2,6-tolylene diisocyanate (TDI) and 44.1 g of ethanol were initially reacted at 40°C for 1 hour, then added with 5 g of a carbodiimidation catalyst and reacted at 75°C for 7 hours to obtain a polycarbodiimide resin (polymerization degree = 5) having blocked ends. This compound was gradually added with 292.4 g of tetrahydrofuran to obtain a solution of Polycarbodiimide Compound 3 (resin concentration: 60% by weight). The carbodiimide equivalent was 183/NCN. When a part of the obtained polycarbodiimide compound (polymerization degree = 5) was examined for water-solubility, this polycarbodiimide compound was hardly dissolved and found to be hydrophobic.

The results of the syntheses of polycarbodiimide compounds obtained above are summarized in Table 2.

Table 2

| Organic compound, Synthesis Example | Functional groups Average no. per molecule | Raw material for blocked end segment | Functional group equivalent | Medium |
|---|---|---|---|---|
| Synthesis Example 1 | Carbodiimide group 5 | Polyoxyethylene monomethyl ether | 318 | Water |
| Synthesis Example 2 | Carbodiimide group 3 | Polyoxyethylene monomethyl ether | 537 | Water |
| Synthesis Example 3 | Carbodiimide group 5 | Ethanol | 183 | THF |

Example 3: Production of Base particle (A)

<1> Production Example 1 of Base Particle

A mixture comprising the following components was charged into a 300-ml flask in a batch, heated at 45°C for about 15 hours on an oil bath with stirring using a stirrer under a nitrogen flow, and a polycarbodiimide compound was thereby reacted to obtain a base particle solution.

| | |
|---|---|
| Solution of Core Particle 1 | 18.0 parts |
| Solution of Polycarbodiimide Compound 1 | 16.6 parts |
| Water | 31.2 parts |
| Methanol | 151.4 parts |

Subsequently, the base particle was repeatedly washed with a mixture of water and methanol (3:7) 3 times and methanol twice or so and filtered by using suction filtration equipment, and then vacuum-dried to obtain Base Particle 1. The shapes of the particles were examined by using SEM (S-2150, Hitachi, Ltd.), and the average particle diameter was found to be 1.76 μm. Further, when the particles were examined by using a Fourier transform infrared spectrophotometer (FT-IR8200PC, Shimadzu Corporation), the absorbance peak of the carbodiimide group was observed at a wavelength of about 2150 (1/cm).

Further, when a part of the particles were dispersed in water as a medium to confirm dispersibility on the basis of particle size distribution (Microtrac 9320HRA, NIKKISO Co., Ltd.), the particle diameter was found to be the same as the result obtained by SEM, which was a particle size suggesting monodispersion.

<2> Production Example 2 of Base Particle

A mixture comprising the following components was charged into a 300-ml flask in a batch, heated at 50°C for about 15 hours on an oil bath with stirring by using a stirrer under a nitrogen flow, and a polycarbodiimide compound was thereby reacted to obtain a base particle solution.

| | |
|---|---|
| Solution of Core Particle 1 | 11.1 parts |
| Solution of Polycarbodiimide Compound 2 | 9.4 parts |
| Water | 28.2 parts |
| Methanol | 74.6 parts |

Subsequently, the base particle was repeatedly washed and filtered with a mixture of water and methanol (3:7) 3 times and methanol twice or so using suction filtration equipment and then vacuum-dried to obtain Base Particle 2. The shapes of the particles were examined by using SEM (S-2150, Hitachi, Ltd.), and the average particle diameter was found to be 1.88 μm. Further, when this particle was examined by using a Fourier transform infrared spectrophotometer (FT-IR8200PC, Shimadzu Corporation), the absorbance peak of the carbodiimide group was observed at a wavelength of about 2150 (1/cm).

Further, when a part of the particles were dispersed in water as a medium to confirm dispersibility on the basis of particle size distribution (Microtrac 9320HRA, NIKKISO Co., Ltd.), the particle diameter was found to be the same as the result obtained by SEM, which was a particle size suggesting monodispersion.

<3> Production Example 3 of Base Particle

A mixture comprising the following components was charged into a 300-ml flask in a batch, heated at 45°C for about 15 hours on an oil bath with stirring by using a stirrer under a nitrogen flow, and a polycarbodiimide compound was thereby reacted to obtain a base particle solution.

| | |
|---|---|
| Solution of Core Particle 2 | 22.0 parts |
| Solution of Polycarbodiimide Compound 1 | 11.1 parts |
| Water | 20.9 parts |
| Methanol | 101.2 parts |

Subsequently, the base particle was repeatedly washed and filtered with a mixture of water and methanol (3:7) 3 times and methanol twice or so using suction filtration equipment and then vacuum-dried to obtain Base Particle 3. The shapes of the particles were examined by using SEM (S-2150, Hitachi, Ltd.), and the average particle diameter was found to be 0.98 μm. Further, when the particles were examined by using a Fourier transform infrared spectrophotometer (FT-IR8200PC, Shimadzu Corporation), the absorbance peak of the carbodiimide group was observed at a wavelength of about 2150 (1/cm).

Further, when a part of the particles were dispersed in water as a medium to confirm dispersibility on the basis of particle size distribution (Microtrac 9320HRA, NIKKISO Co., Ltd.), the particle diameter was found to be the same as the result obtained by SEM, which was a particle size suggesting monodispersion.

<4> Production Example 4 of Base Particle

A mixture comprising the following components was charged into a 300-ml flask in a batch, heated at 50°C for about 15 hours on an oil bath with stirring using a stirrer under a nitrogen flow, and a polycarbodiimide compound was thereby reacted to obtain a base particle solution.

| | |
|---|---|
| Solution of Core Particle 2 | 22.1 parts |
| Solution of Polycarbodiimide Compound 2 | 12.5 parts |
| Water | 37.8 parts |
| Methanol | 99.8 parts |

Subsequently, the base particle was repeatedly washed and filtered with a mixture of water and methanol (3:7) 3 times and methanol twice or so using suction filtration equipment and then vacuum-dried to obtain Base Particle 4. The shapes of the particles were examined by using SEM (S-2150, Hitachi, Ltd.), and the average particle diameter was found to be 1.06 μm. Further, when the particles were examined by using a Fourier transform infrared spectrophotometer (FT-IR8200PC, Shimadzu Corporation), an absorbance peak of the carbodiimide group was observed at a wavelength of about 2150 (1/cm).

Further, when a part of the particles were dispersed in water as a medium to confirm dispersibility on the basis of particle size distribution (Microtrac 9320HRA, NIKKISO Co., Ltd.), the particle diameter was found to be the same as the result obtained by SEM, which was a particle size suggesting monodispersion.

<5> Production Example 5 of Base Particle

In accordance with the method described in Japanese Patent Laid-open No. 8-23975, Example 6, a mixture comprising the following components was charged into a 300-ml flask in a batch, and immersion was performed for about 30 minutes with stirring using a stirrer.

| | |
|---|---|
| Cross-linked particles (main component: divinyl benzene)* | 5.0 parts |
| Solution of Polycarbodiimide Compound 3 | 16.7 parts |
| THF | 83.3 parts |

*: Cross-linked particles (SP-2095 produced by Sekisui fine Chemical Co. Ltd., average particle diameter: 9.5 μm, CV value: 4.7%)

Subsequently, the aforementioned mixture was filtered by using suction filtration equipment and dried by using a drier at temperature of 60°C for about 3 hours to obtain polycarbodiimide compound-coated particles.

When the particles were examined by using a Fourier transform infrared spectrophotometer (FT-IR8200PC, Shimadzu Corporation), an absorbance peak of the carbodiimide group was observed at a wavelength of about 2150 (1/cm).

However, when a part of the particles were dispersed in water as a medium to confirm dispersibility on the basis of particle size distribution (Microtrac 9320HRA, NIKKISO Co., Ltd.), a distribution represented by one peak curve having a larger width and longer distribution tails compared with particle diameter distribution of the base particles of Production Examples 1 to 4 was obtained. When the shapes of the particles were examined by using SEM (S-2150, Hitachi, Ltd.), the average particle diameter was found to be 17.06 μm, and some aggregated particles were observed. When the average particle diameter was calculated, aggregated particles were assumed as one particle, and an average value of the largest particle diameter and the smallest diameter obtained with SEM was calculated as a particle diameter.

The results of the above production of base particles are summarized in Table 3.

Table 3

| Base Particle Production Example | Core particle used | Addition amount of Organic compound A (equivalent ratio as to functional group) | Synthesis temperature (°C) | Dispersibility in water as medium |
|---|---|---|---|---|
| 1 | Production Example 1 | 3 | 45 | ○ |
| 2 | Production Example 1 | 2 | 50 | ○ |
| 3 | Production Example 2 | 3 | 45 | ○ |
| 4 | Production Example 2 | 2 | 50 | ○ |

Table continued

| Base Particle Production Example | Core particle used | Addition amount of Organic compound A (equivalent ratio as to functional group) | Synthesis temperature (°C) | Dispersibility in water as medium |
|---|---|---|---|---|
| 5 | - | - | - | Δ |
| ○: Monodispersed base particles<br>Δ: Partially monodispersed aggregated base particles<br>×: Base particles mostly consisting of aggregated particles | | | | |

Example 4: Evaluation of core particles and base particles

<1> Evaluation Test 1

Base particles 1 to 5 produced in Example 3 and the core particles used in production of these particles were each photographed by using SEM with a magnification enabling measurement (x 100 to 10,000), and the major axis and the minor axis were randomly measured 15 times for one particle. This measurement was repeatedly performed for randomly selected particles (n = 100). Then, the average values were calculated for the measured results, and a spherical particle exponential mean (major axis/minor axis) was calculated. The results are shown in Table 4.

Table 4

| Base particle Production Example | Spherical particle exponential mean for core particles | Spherical particle exponential mean for base particles | Monodispersion, sphericalness |
|---|---|---|---|
| 1 | 1.04 | 1.03 | ○ |
| 2 | 1.04 | 1.05 | ○ |
| 3 | 1.05 | 1.05 | ○ |
| 4 | 1.05 | 1.06 | ○ |
| ○: Highly precise particles of which precipitation rate, particle surface area and addition amount of biologically active substance can be equalized<br>×: Poor precision particles of which precipitation rate, particle surface area and addition amount of biologically active substance are difficult to be equalized | | | |

<2> Evaluation Test 2

The base particles produced in Example 3 and the core particles used for production of these particles were each photographed by using SEM (x 100 to 10,000), the particle diameters were measured for randomly selected particles ($n_1$ = 500), and the average particle diameter was calculated. Then, the average thickness diameter (L) of the carbodiimide compound layer was calculated according to the following equation. The results are shown in Table 5.

$$L = (L_2 - L_1)/2$$

$L_1$ is the average particle diameter of the experimentally produced core particles ($A_1$).
$L_2$ is the average particle diameter of the experimentally produced base particles (A).

Table 5

| Base Particle Production Example | Average particle diameter of core particles (μm) | Average particle diameter of base particles (μm) | L (μm) |
|---|---|---|---|
| 1 | 1.42 | 1.76 | 0.17 |
| 2 | 1.42 | 1.88 | 0.23 |
| 3 | 0.78 | 0.98 | 0.10 |
| 4 | 0.78 | 1.06 | 0.14 |
| 5 | 9.5 | 17.06 | *** |
| ***: Numerical measurement was impossible due to aggregation of particles | | | |

<3> Evaluation Test 3

CV values and CV ratios were calculated from the measurement results for the base particles and the core particles obtained in the aforementioned evaluation. The results are shown in Table 6.

Table 6

| Base Particle Production Example | $CV_1$ value (%) of core particle | $CV_2$ value (%) of base particle | $CV_a$ ratio CV1/CV2 |
|---|---|---|---|
| 1 | 4.58 | 4.32 | 1.06 |
| 2 | 4.58 | 4.26 | 1.08 |
| 3 | 6.06 | 5.94 | 1.02 |
| 4 | 6.06 | 5.82 | 1.04 |
| 5 | 4.7 | 33.54 | 0.14 |

From the results of Evaluation Tests 1 to 3 described above, it was confirmed that the base particles having Organic compound A used in the examples of the present invention were base particles having a layer of Organic compound A on the surface layer portions of the core particles, and were spherical particles having relatively even particle sizes.

Further, Base Particles obtained in the above production example 1 to 4 were dispersed in water as a solvent by using known dispersion equipment to form 1 weight % particle aqueous dispersions as solutions of Base Particle 1 to 4. The solutions of Base Particles 1 to 4 were examined by using a particle size distribution analyzer (Microtrac 9320HRA, NIKKISO Co., Ltd.), and it was found that the particles had average particle diameters comparable to those of the aforementioned particles, and as for distribution, they were monodispersed particles of which distribution was represented by a sharp one-peak curve.

Example 5

<1> Production of device

Oligonucleotides (30-mers) having the nucleotide sequences of SEQ ID NOS: 1, 2 and 3 were synthesized by using an oligonucleotide synthesizer (Perkin-Elmer Applied Biosystems) in a conventional manner. The oligonucleotide of SEQ ID NO: 1 was biotinylated at the 5' end. The oligonucleotide of SEQ ID NO: 2 was complementary to a biotinylated probe (SEQ ID NO: 4), and the oligonucleotide of SEQ ID NO: 3 differed from the oligonucleotide of SEQ ID NO: 2 by one nucleotide and thus was not complementary thereto. These oligonucleotides were each dissolved in 3 × SSC at 100 pmol/μl.

Each of the aforementioned oligonucleotide solutions was mixed with the solution of Base Particle 1 mentioned above in a quartz cell. Then, the mixture was irradiated with an ultraviolet ray at 1200 mJ/$cm^2$ from 16 cm away by using Uvstratalinker 2400 (STRATAGENE). The irradiation time was 480 seconds. Then, the base particles were washed by shaking in water for 30 minutes and dried to obtain devices.

<2> Hybridization

The aforementioned devices and 60 μl of hybridization solution (Arrayit UniHyb (TeleCHem International, Inc.) containing 3 pmol of the biotinylated probe (SEQ ID NO: 4, 262 bp) were mixed in an Eppendorf tube and heated at 45°C for 2 hours by using a drier. The oligonucleotide of SEQ ID NO: 2 contained a sequence complementary to the biotinylated probe (SEQ ID NO: 4).

Following the aforementioned hybridization, post-hybridization washing was performed under the following conditions to remove the biotinylated probes non-specifically adsorbed on the devices.

[Conditions for post-hybridization washing]

1) 2 × SSC, 0.1% SDS, room temperature, 5 minutes, twice
2) 0.2 × SSC, 0.1% SDS, 40°C, 5 minutes, twice
3) 2 × SSC, room temperature, 1 minute, 3 times

In an amount of 1.5 ml of a blocking solution containing lactoproteins (Block Ace, Snow Brand Milk Products Co., Ltd.) was placed on the devices to perform blocking at room temperature for 30 minutes. The blocking solution was removed, and then 1.5 ml of a streptavidin/alkaline phosphatase conjugate solution (VECTOR) was placed and reacted at room temperature for 30 minutes. Subsequently, the devices were immersed in a TBST solution (50 mM Tris-HCl (pH 7.5), 0.15 M NaCl, 0.05% Tween 20) and shaken for 5 minutes to remove unreacted conjugates. Finally, the devices were added with 1.5 ml of a substrate solution (TMB) and left for 30 minutes to allow color development reaction.

The results are shown in Table 7. The signals of the particles on which the oligonucleotide of SEQ ID NO: 2 was immobilized represent the amount of the immobilized oligonucleotide. The signals of the particles on which the oligonucleotide of SEQ ID NO: 3 was immobilized represent intensity of hybridization.

Comparative Example 1

<1> Production of device

A solution of Base Particle (base particles produced according to the method described in Japanese Patent Laid-open No. 8-23975, Example 6) produced in Example 3, Production Example 5 of Base Particle and each of the oligonucleotide solutions prepared in Example 5 were mixed in a quartz cell. Then, the mixture was irradiated with an ultraviolet ray at 1200 mJ/cm$^2$ from 16 cm away by using Uvstratalinker 2400 (STRATAGENE). The irradiation time was 480 seconds. Then, the base particles were washed in water for 30 minutes with shaking and then dried to obtain devices.

<2> Hybridization

The aforementioned devices and 60 μl of hybridization solution (Arrayit UniHyb, TeleCHem International, Inc.) containing 3 pmol of biotinylated probe (SEQ ID NO: 4, 262 bp) were mixed in an Eppendorf tube and heated at 45°C for 2 hours by using a drier.

Following the aforementioned hybridization, hybridization was detected in the same manner as in Example 5. The results are shown in Table 7.

Table 7

| | Immobilized oligonucleotide | | |
|---|---|---|---|
| | SEQ ID NO: 1 | SEQ ID NO: 2 | SEQ ID NO: 3 |
| Example 5 | ◎ | ◎ | × |
| Comparative Example 1 | Δ | Δ | × |

◎: Very clear signals were observed with very high sensitivity.

○: Clear signals were observed with high sensitivity.

Δ: Signals were observed with low sensitivity or unclearly.

×: No signal was observed.

As demonstrated by the results shown in Table 7, it was found that the oligonucleotides were reliably immobilized on the base particles in the devices of Example 5. Further, in the devices of Example 5, the hybridization signals were also clearly observed. No signal was observed from the oligonucleotide of SEQ ID NO: 3. On the other hand, in the devices of Comparative Example 1, the signals from the immobilized oligonucleotides and hybridization signal were observed unclearly with low sensitivity, and therefore it is considered that the carbodiimide compound was fallen off from the base particles. Therefore, it is considered that, in the devices of Example 5, the carbodiimide compound was prevented from falling off from the base particles by the formation of covalent bonds between the base particles and the carbodiimide compound, and as a result, a clear signal could be obtained with high sensitivity.

When devices were produced by immobilizing oligonucleotides on the base particles using the solution of Base Particles 2 to 4, and hybridization was detected in a similar manner, results similar to those of Example 5 were obtained.

Example 6: Evaluation of dispersibility of devices

The devices obtained in Example 5 and Comparative Example 1 were diluted with water, and dispersibility was confirmed by using a particle size distribution analyzer (Microtrac 9320HRA, NIKKISO Co., Ltd.).

As a result, the devices of Example 5 were particles showing monodispersion distribution similar to that of Base Particle 1, and no change indicating a different distribution was observed. On the other hand, the distribution of the devices immobilized with the oligonucleotide of Comparative Example 1 was wider than the particle diameter distribution of the used core particles, and it was represented by a one-peak curve with long distribution tails.

Further, as confirmed by using SEM (x 100 to 10,000), aggregated particles and deformed particles (including the shapes of base particles) were not observed among the devices of Example 5, whereas some aggregated particles were observed among the devices of Comparative Example 1. These results are shown in Table 8, and the device diameters, CV values and CV ratios are shown in Table 9.

Table 8

| | Dispersibility based on particle size distribution | Aggregation/deformation observed by SEM |
|---|---|---|
| Device of Example 5 | A | No aggregated/deformed particles |
| Device of Comparative Example 1 | B | Aggregated particles |
| A: Monodispersed devices having a particle diameter similar to that of the base particles<br>B: Devices a part of which had a particle diameter similar to that of the base particles, but which had a wide distribution range<br>C: Devices not having a particle diameter similar to that of the base particles and having a wide distribution range. | | |

Table 9

| | Device average particle diameter (μm) | Device $CV_3$ value (%) | $CV_b$ ratio $CV_1/CV_3$ | $CV_c$ ratio $CV_2/CV_3$ |
|---|---|---|---|---|
| Example 5 | 1.81 | 4.64 | 0.99 | 0.93 |
| Comparative Example 1* | 21.72 | 64.38 | 0.07 | 0.52 |
| *: Aggregated particles were assumed as one particle, and average of the major axis and the minor axis of the particles was obtained by using SEM as the diameter of the particle. | | | | |

This confirmed that the devices of the present invention had favorable solution dispersibility and high performance.

When dispersibility was examined for the devices produced by using Base Particles 2 to 4, it was confirmed that they showed favorable particle size distribution and shape observed by using SEM and exhibited favorable dispersibility in a solution.

Industrial Applicability

The device of the present invention shows good stability for dispersion in a solution and can be suitably used for detection or measurement of a biologically active substance or for therapeutic treatment.

**Claims**

1. A biologically active substance-immobilized device, which comprises a base particle comprising a core particle and an organic compound having two or more hydrophilic groups and immobilized on the core particle by a chemical bond and a biologically active substance bonded to the base particle via the organic compound.

2. The device according to claim 1, which is used in an aqueous medium.

3. The device according to claim 1 or 2, wherein the base particle has an average particle diameter of 0.01 to 100 μm.

4. The device according to any one of claims 1 to 3, wherein the base particle has a spherical or substantially spherical shape.

5. The device according to any one of claims 1 to 4, wherein at least one of $CV_b$ ratio and $CV_c$ ratio defined by the following equations is 0.6 to 3.0:

$$CV_b \text{ ratio} = CV_1/CV_3$$

$$CV_c \text{ ratio} = CV_2/CV_3$$

$$CV_1 = (\text{Standard deviation of core particle diameter/Average core particle diameter}) \times 100$$

$$CV_2 = (\text{Standard deviation of base particle diameter/Average base particle diameter}) \times 100$$

$$CV_3 = (\text{Standard deviation of device diameter/average device particle diameter}) \times 100$$

**6.** The device according to any one of claims 1 to 5, wherein the core particle and the biologically active substance are bonded by a reaction with a functional group selected from carbodiimide group, ester group, carbonate group, epoxy group and oxazoline group.

**7.** The device according to any one of claims 1 to 6, wherein the organic compound is a compound represented by the following formula:

$$A_x—(R—X)_n—R—A_y \qquad (I)$$

wherein $A_x$ and $A_y$ independently represent a segment having a functional group that exhibits hydrophilicity and may be identical or different, R independently represents an organic group of two or more valences,X independently represents carbodiimide group, epoxy group or oxazoline group, and n is an integer of 2 to 80, preferably 2 to 40.

**8.** The device according to any one of claims 1 to 7, wherein the biologically active substance is selected from a nucleic acid, protein, hapten and saccharide.

**9.** The device according to any one of claims 1 to 8, which is for detecting or measuring a second biologically active substance contained in a sample by using a specific bond of the biologically active substance and the second biologically active substance in the sample.

**10.** The device according to any one of claims 1 to 8, wherein the biologically active substance is an agent for therapeutic treatment of a disease.

**INTERNATIONAL SEARCH REPORT**

International application No.
PCT/JP2004/005498

A. CLASSIFICATION OF SUBJECT MATTER
Int.Cl$^7$ G01N33/547, G01N33/543, C12N15/00, C12Q1/68

According to International Patent Classification (IPC) or to both national classification and IPC

B. FIELDS SEARCHED

Minimum documentation searched (classification system followed by classification symbols)
Int.Cl$^7$ G01N33/547, G01N33/543, C12N15/00, C12Q1/68

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2004 |
| Kokai Jitsuyo Shinan Koho | 1971-2004 | Jitsuyo Shinan Toroku Koho | 1996-2004 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X/A | JP 2000-146978 A (Nisshinbo Industries, Inc.), 26 May, 2000 (26.05.00), Claims; Par. Nos. [0028], [0029], [0041], [0043] & EP 1001267 A & US 2001/0055762 A | 1-4,6-10/5 |
| X/ A | JP 58-47258 A (E.I. Du Pont De Nemours & Co.), 18 March, 1983 (18.03.83), Claims; page 9, lower right column, line 8 to page 10, upper left column, line 7; page 10, lower left column, line 7 to lower right column, line 8 & EP 073611 A & US 4401765 A | 1-4,6,8-10/ 5,7 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>23 June, 2004 (23.06.04) | Date of mailing of the international search report<br>13 July, 2004 (13.07.04) |
| Name and mailing address of the ISA/<br>Japanese Patent Office | Authorized officer |
| Facsimile No. | Telephone No. |

Form PCT/ISA/210 (second sheet) (January 2004)